# EUROPEAN PATENT APPLICATION

(11) **EP 4 209 181 A1**
(43) Date of publication of application: **12.07.2023**
(21) Application number: 21863618.1
(22) Date of filing: 01.09.2021
(51) Int. Cl.: A61B 17/00, A61B 34/10

(54) **POSTEROMEDIAL STRUCTURE, POSTEROLATERAL STRUCTURE, AND MEDIAL PATELLOFEMORAL LIGAMENT RECONSTRUCTION POSITIONING SYSTEM AND METHOD**

(30) Priority: 02.09.2020 CN 202010912346; 31.12.2020 CN 202011641992
(71) Applicant: Shanghai Droidmeds Medical Co., Ltd, Shanghai 200135 (CN)
(72) Inventor: WANG, Shaobai, Shanghai 201615 (CN); ZHAO, Jinzhong, Shanghai 201615 (CN); HOU, Yao, Shanghai 201615 (CN); JIANG, Jia, Shanghai 201615 (CN); BIAN, Zhiqi, Shanghai 201615 (CN); ZHOU, Wujian, Shanghai 201615 (CN); SHEN, Hui, Shanghai 201615 (CN)
(74) Representative: Argyma
(86) International application number: PCT/CN2021/115867
(87) International publication number: WO 2022/048554

(57) **Abstract**

Provided is a positioning system for posteromedial structure, posterolateral structure and medial patellofemoral ligament reconstruction, which relates to the technical field of ligament reconstruction. The system includes: a reconstruction channel coordinate system establishment module for establishing any one or more of a femoral coordinate system, a tibial coordinate system and a patellar coordinate system; a reconstruction channel bone feature point obtainment module for selecting femoral feature points, tibial feature points, fibular feature points or patellar feature points; and a reconstruction channel position determination module for determining a femoral point, a tibial point, a fibular point and a patellar point during reconstruction according to a preset reconstruction algorithm, and further determining a specific channel position of a posteromedial structure, posterolateral structure or medial patellofemoral ligament reconstruction channel by means of the femoral point, the tibial point, the fibular point and the patellar point. The present disclosure provides a standard method for selecting the femoral point, tibial point, fibular point or patellar point during tunnel reconstruction, which can make the specific path of the reconstruction tunnel more accurate.

## Description

### BACKGROUND OF THE INVENTIONFIELD OF THE INVENTION

The present disclosure relates to the technical field of posteromedial structure, posterolateral structure and medial patellofemoral ligament reconstruction, and particularly to a positioning system and method for posteromedial structure, posterolateral structure and medial patellofemoral ligament reconstruction.

### DESCRIPTION OF THE PRIOR ART

Ligament injury to a knee, one of the most complex joints of a human body, is a common disease in sport medicine. In order to prevent knee instability from further degenerating into osteoarthritis or other adverse consequences, it is necessary to reconstruct a ruptured ligament in time. Knee ligament reconstruction generally involves diagnostic arthroscopy, cutting and preparation of a ligament graft, establishment of a bone tunnel with a ligament anatomical attachment point as an entrance and exit, and grafting and fixation of the graft, among which the establishment of the bone tunnel for fixing a reconstructed ligament is vital and difficult during surgery.

In the prior art, during establishment of the bone tunnel, selection of points on a femur and a tibia generally relies on the experience of a doctor, which is time-consuming and labor-consuming, and the surgery is likely to fail due to inexperience.

### SUMMARY OF THE INVENTION

In order to solve the above problems, an objective of the present disclosure is to provide a positioning system and method for posteromedial structure, posterolateral structure and medial patellofemoral ligament reconstruction, which provide a standard method for selecting the fibular point, the femoral point and the tibial point during posteromedial structure, posterolateral structure and medial patellofemoral ligament channel reconstruction. Through the method in the present disclosure, a specific path of a formed reconstruction tunnel may be more accurate, and a success rate of subsequent ligament reconstruction surgery by means of the reconstruction channel in the present disclosure may be increased.

The above objective of the present disclosure is realized through technical solutions as follows:
A channel positioning system for posteromedial structure, posterolateral structure and medial patellofemoral ligament reconstruction includes:
a reconstruction channel point collecting and modeling device for determining a specific channel position of a reconstruction channel and specifically including:
a reconstruction channel coordinate system establishment module for establishing any one or more of a femoral coordinate system, a tibial coordinate system and a patellar coordinate system;
a reconstruction channel bone feature point obtainment module for selecting femoral feature points, tibial feature points, fibular feature points or patellar feature points by means of a probe under an arthroscope; and
a reconstruction channel position determination module for determining a femoral point, a tibial point, a fibular point and a patellar point during reconstruction according to a preset reconstruction algorithm on the basis of the femoral feature points, the tibial feature points, the fibular fe ature points and the patellar feature points, and further determining a specific channel position of a posteromedial structure, posterolateral structure or medial patellofemoral ligament reconstruction channel by means of the femoral point, the tibial point, the fibular point and the patellar point.

The positioning system for posteromedial structure, posterolateral structure and medial patellofemoral ligament reconstruction further includes:
a reconstruction channel mechanical arm positioning device for being connected to the reconstruction channel point collecting and modeling device to carry out physically assisted positioning on the reconstruction channel according to a determined specific channel position of the reconstruction channel, so as to guide posteromedial structure, posterolateral structure and medial patellofemoral ligament reconstruction surgery.

The positioning system for posteromedial structure, posterolateral structure and medial patellofemoral ligament reconstruction further includes:
a reconstruction channel arthroscopy device for observing an inner structure of a joint to undergo surgery, and monitoring posteromedial structure, posterolateral structure and medial patellofemoral ligament reconstruction surgery processes in real time, where the reconstruction channel point collecting and modeling device is used for positioning the specific channel position of the reconstruction channel according to the inner structure of the joint to undergo surgery.

A positioning method for posteromedial structure, posterolateral structure and medial patellofemoral ligament reconstruction includes steps as follows:
S1: establishing any one or more of a femoral coordinate system, a tibial coordinate system and a patellar coordinate system;
S2: selecting femoral feature points, tibial feature points, fibular feature points or patellar feature points by means of a probe under an arthroscope; and
S3: determining a femoral point, a tibial point, a fibular point or a patellar point during reconstruction according to a preset reconstruction algorithm on the basis of the femoral feature points, the tibial feature points, the fibular feature points or the patellar feature points, and further determining a specific channel position of a posteromedial structure, posterolateral structure or medial patellofemoral ligament reconstruction channel by means of the femoral point, the tibial point, the fibular point or the patellar point.

An intelligent control method for ligament reconstruction on the basis of the positioning system for posteromedial structure, posterolateral structure and medial patellofemoral ligament reconstruction includes steps as follows:
S100: collecting several position points within a sight range of a reconstruction channel arthroscopy device to form a sampling point set, and establishing a three-dimensional coordinate system according to the sampling point set;
S200: selecting several bone feature points from the three-dimensional coordinate system, and establishing a tunnel position path model according to the bone feature points;
S300: determining a position of a tunnel inner orifice according to the tunnel position path model;
S400: positioning a hollow sleeve by means of a mechanical arm according to the position of the tunnel inner orifice, so as to align an inner cavity of the hollow sleeve with the tunnel inner orifice; and
S500: establishing a tunnel by means of a tool under guidance of the hollow sleeve.

Compared with the prior art, the present disclosure has at least one type of beneficial effects as follows:
(1) A positioning system for posteromedial structure, posterolateral structure and medial patellofemoral ligament reconstruction is provided and includes: a reconstruction channel point collecting and modeling device for determining a specific channel position of a reconstruction channel and specifically including: a reconstruction channel coordinate system establishment module for establishing any one or more of a femoral coordinate system, a tibial coordinate system and a patellar coordinate system; a reconstruction channel bone feature point obtainment module for selecting femoral feature points, tibial feature points, fibular feature points or patellar feature points by means of a probe under an arthroscope; and a reconstruction channel position determination module for determining a femoral point, a tibial point, a fibular point and a patellar point during reconstruction according to a preset reconstruction algorithm on the basis of the femoral feature points, the tibial feature points, the fibular feature points and the patellar feature points, and further determining a specific channel position of a posteromedial structure, posterolateral structure or medial patellofemoral ligament reconstruction channel by means of the femoral point, the tibial point, the fibular point and the patellar point. According to the above technical solution, a standard method for selecting the femoral point, the tibial point, the fibular point and the patellar point during posteromedial structure, posterolateral structure and medial patellofemoral ligament channel reconstruction is provided. Through the method in the present disclosure, a specific path of a formed reconstruction tunnel may be more accurate, and a success rate of surgery may be increased.
(2) The system in the present disclosure further includes a posteromedial structure, posterolateral structure and medial patellofemoral ligament reconstruction mechanical arm positioning device and arthroscopy device besides the posteromedial structure, posterolateral structure and medial patellofemoral ligament point collecting and modeling device. According to the above technical solution, by means of the arthroscopy device, an inner structure of a joint may be observed, and the whole process of surgery may be accurately observed; by means of the point collecting and modeling device, a position of a tunnel in the joint is determined, and a reconstruction tunnel is established to simulate a tunnel path; and by means of the mechanical arm positioning device, the tunnel path is subjected to physical positioning, so as to guide surgery.

### BRIEF DESCRIPTION OF DRAWINGS

Various other advantages and benefits will become apparent to those of ordinary skill in the art upon reading the following detailed description of the preferred embodiments. Accompanying drawings are merely used for the objective of illustrating preferred embodiments, and are not to be regarded as limiting the present disclosure.
FIG. 1 is an overall structural diagram of a positioning system for posteromedial structure, posterolateral structure and medial patellofemoral ligament reconstruction according to Example 1 of the present disclosure;
FIG. 2 is an overall schematic diagram of a positioning system for posteromedial structure, posterolateral structure and medial patellofemoral ligament reconstruction according to the present disclosure;
FIG. 3 is a schematic diagram of a femoral coordinate system according to Example 1 of the present disclosure:
FIG. 4 is a schematic diagram of a tibial coordinate system according to Example 1 of the present disclosure;
FIG. 5 is a schematic diagram of a patellar coordinate system according to Example 1 of the present disclosure;
FIG. 6 is a schematic diagram of selected points on a femoral lateral condyle according to Example 2 of the present disclosure;
FIG. 7 is a schematic diagram of a selected point at a tibial medial side according to Example 2 of the present disclosure;
FIG. 8 is a schematic diagram of a selected point at a tibial anterior side according to Example 2 of the present disclosure;
FIG. 9 is a schematic diagram of selected points on a fibula according to Example 2 of the present disclosure;
FIG. 10 is a schematic diagram of selected points on a patella according to Example 2 of the present disclosure;

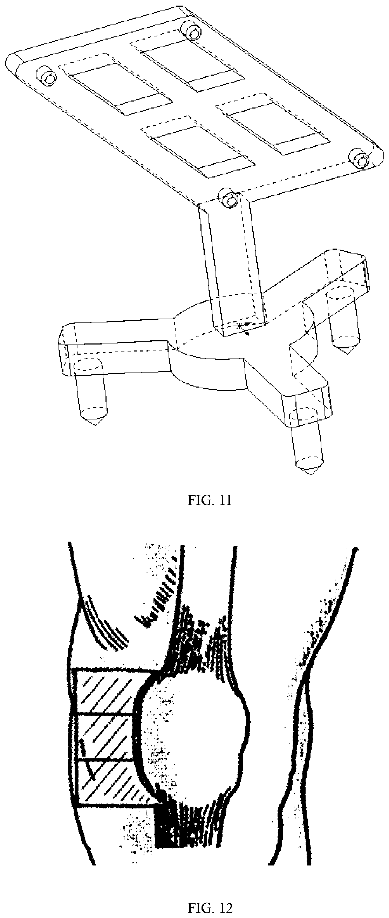

FIG. 11 is a schematic diagram of a chuck-type patellar marking device according to Example 4 of the present disclosure;
FIG. 12 is a schematic diagram of a patella divided into three equal parts from top to bottom according to Example 4 of the present disclosure.

### DETAILED DESCRIPTION OF THE EMBODIMENTS

In order to make objectives, technical solutions, and advantages of examples of the present disclosure clearer, technical solutions of examples of the present disclosure will be clearly and completely described below in combination with accompanying drawings in examples of the present disclosure. Apparently, the described examples are part of the present disclosure, rather than all of them. On the basis of examples of the present disclosure, all other examples obtained by those of ordinary skill in the art without making creative efforts fall within the scope of protection of the present disclosure.

It can be understood by those skilled in the art that singular forms "alan", "one", "the" and "this" used herein can also include plural forms unless expressly stated. It should be further understood that word "comprise/include" used in the description of the present disclosure means presence of stated features, integers, steps, operations, elements and/or assemblies, but does not exclude the presence or addition of one or more other features, integers, steps, operations, elements, assemblies and/or groups thereof.

### Example 1

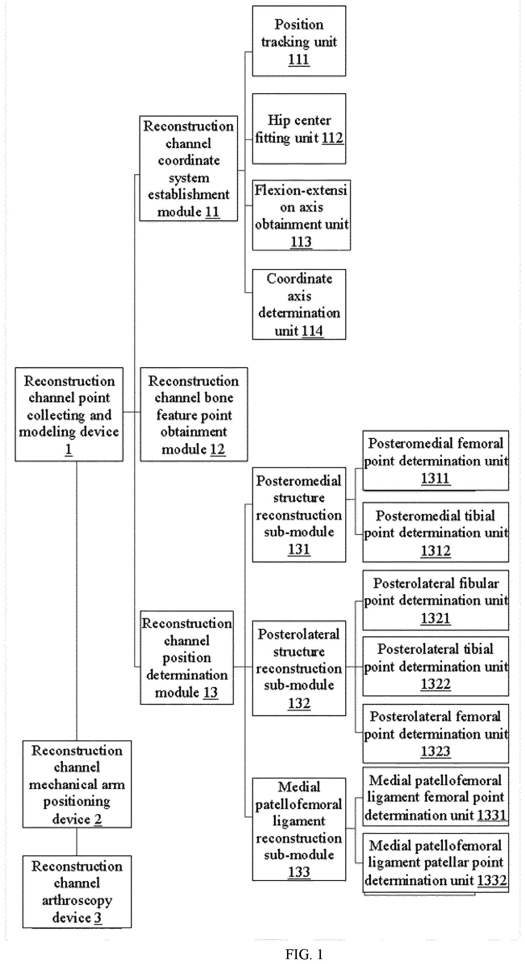

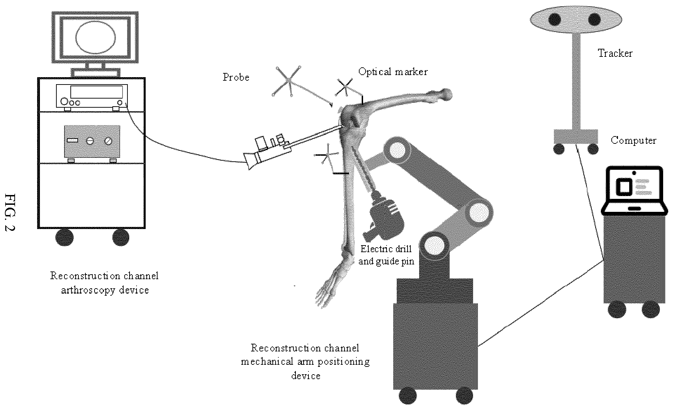

As shown in FIGs. 1 and 2, the example provides a positioning system for posteromedial structure, posterolateral structure and medial patellofemoral ligament reconstruction. The system includes:
a reconstruction channel point collecting and modeling device 1 for determining a specific channel position of a reconstruction channel and specifically including:
a reconstruction channel coordinate system establishment module 11 for establishing any one or more of a femoral coordinate system, a tibial coordinate system and a patellar coordinate system.

Specifically, in order to conveniently select a femoral point, a tibial point and a patellar point of a tunnel in a tunnel reconstruction process subsequently, and in the case that a position of a femur, a position of a tibia or a position of a patella is not fixed, the situation that a coordinate system is not unified due to rotation of the bone, such that points are difficult to select may not occur. It is required to establish corresponding coordinate systems for the femur, the tibia or the patella respectively in advance, and the femoral point is selected according to the femoral coordinate system, the tibial point is selected according to the tibial coordinate system, and the patellar point is selected according to the patellar coordinate system subsequently.

It should be noted that a specific method for establishing a femoral coordinate system, a tibial coordinate system and a patellar coordinate system is not limited in the present disclosure, and it is only required to provide a reference for selection of the femoral point, the tibial point and the patellar point after the coordinate systems are established.

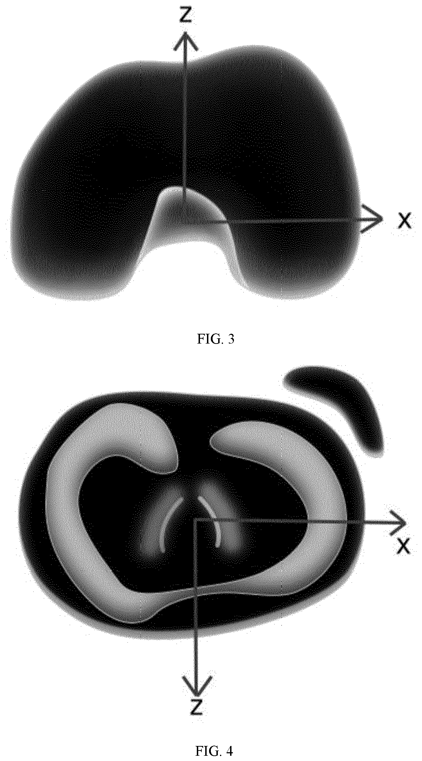

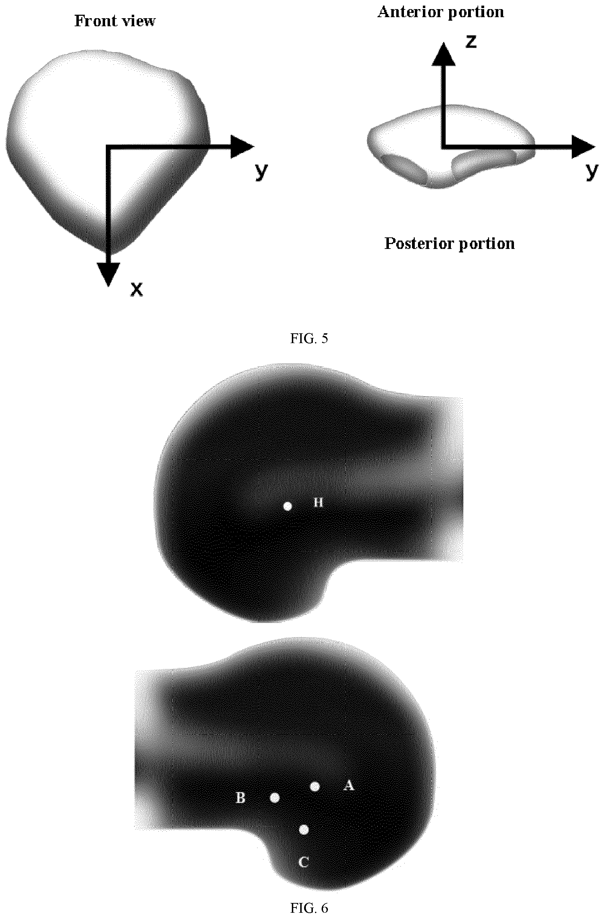

As shown in FIG. 3 (a femoral coordinate system), FIG. 4 (a tibial coordinate system) and FIG. 5 (a patellar coordinate system), a module corresponding to the specific method for establishing a femoral coordinate system, a tibial coordinate system and a patellar coordinate system is listed. The module specifically includes:
a position tracking unit 111 for fixing an optical femoral marker and an optical tibial marker on a femur and a tibia respectively, and tracking a position of the femur and a position of the tibia in real time by an optical tracker;
a hip center fitting unit 112 for obtaining a femoral position data set of the optical femoral marker under the optical tracker, and fitting the femoral position data set to compute a hip midpoint;
a flexion-extension axis obtainment unit 113 for obtaining a tibial position data set of the optical tibial marker under the optical tracker, fitting the tibial position data set to obtain a plane, taking a normal line of the plane as a flexion-extension axis of the tibia, and taking the flexion-extension axis as a femoral x-axis of the femoral coordinate system and a tibial x-axis of the tibial coordinate system;
a coordinate axis determination unit 114 for establishing the femoral coordinate system, the tibial coordinate system or the patellar coordinate system.

A reconstruction channel bone feature point obtainment module 12 is used for selecting femoral feature points, tibial feature points, fibular feature points or patellar feature points by means of a probe under an arthroscope.

Specifically, in this step, before the femoral point, the tibial point, the fibular point or the patellar point for tunnel reconstruction are selected, it is required to select the plurality of bone feature points serving as reference points on the femur, the tibia, the fibula or the patella, and subsequently the femoral point, the tibial point, the fibular point or the patellar point are selected according to the selected bone feature points.

A reconstruction channel position determination module 13 is used for determining a femoral point, a tibial point, a fibular point and a patellar point during reconstruction according to a preset reconstruction algorithm on the basis of the femoral feature points, the tibial feature points, the fibular feature points and the patellar feature points, and further determining a specific channel position of a posteromedial structure, posterolateral structure or medial patellofemoral ligament reconstruction channel by means of the femoral point, the tibial point, the fibular point and the patellar point.

Specifically, the reconstruction channel position determination module 13 specifically includes any one or more of sub-modules as follows:
a posteromedial structure reconstruction sub-module 131 for executing a posteromedial structure reconstruction algorithm, so as to reconstruct a specific channel position of a posteromedial structure reconstruction channel;
a posterolateral structure reconstruction sub-module 132 for executing a posterolateral structure reconstruction algorithm, so as to reconstruct a specific channel position of a posterolateral structure reconstruction channel;
a medial patellofemoral ligament reconstruction sub-module 133 for executing a medial patellofemoral ligament reconstruction algorithm, so as to reconstruct a specific channel position of a medial patellofemoral ligament reconstruction channel.

### (1) Posteromedial structure reconstruction sub-module 131

A posteromedial femoral point determination unit 1311 is used for determining the femoral point for posteromedial structure reconstruction, which specifically includes: use a method of moving a medial epicondyle top point on the femur by a first preset distance in a positive direction of a y -axis and a second preset distance in a negative direction of a z-axis of the femoral coordinate system as a double-tunnel femoral point selection method, use a method of moving the medial epicondyle top point on the femur by preset distances in a positive direction of a y-axis and in a negative direction of a z-axis of the femoral coordinate system as a single-tunnel femoral point selection method, or determining the femoral point including an adductor tubercle proximal side and a femoral medial central established proximal side.

A posteromedial tibial point determination unit 1312 is used for determining the tibial point for posteromedial structure reconstruction, which specifically includes: move a posteromedial tibial plateau inferior portion by a preset distance in a negative direction of a y-axis of the tibial coordinate system to point to a tibial anterolateral side, and moving a Gerdy tubercle distal portion and a posterior medial crest constriction point anterior side by preset distances in a negative direction of a z-axis of the tibial coordinate system to point to a tibial lateral surface or a medial crest superior portion and a constriction point.

Further, in the case of the posteromedial femoral point determination unit 1311, a method of moving a medial epicondyle top point on the femur by a first preset distance in a positive direction of a y - axis and a second preset distance in a negative direction of a z-axis of the femoral coordinate system is used as a double-tunnel femoral point selection method, where the first preset distance ranges from 4 mm to 5mm, and the second preset distance ranges from 12 mm to 14 mm; and a method of moving the medial epicondyle top point on the femur by preset distances in a positive direction of a y-axis, and in a negative direction of a z-axis of the femoral coordinate system is used as a single-tunnel femoral point selection method, where the preset distances each range from 8 mm to 10 mm.

In the case of the posteromedial tibial point determination unit 1312, a posteromedial tibial plateau inferior portion is moved by a preset distance in a negative direction of a y-axis of the tibial coordinate system to point to a tibial anterolateral side, where the preset distance ranges from 9 mm to 11mm; and a Gerdy tubercle distal portion and a posterior medial crest constriction point anterior side are moved by preset distances in a negative direction of a z-axis of the tibial coordinate system to point to a tibial lateral surface or a medial crest superior portion and a constriction point, where the preset distances each range from 9 mm to 11 mm.

### (2) Posterolateral structure reconstruction sub-module 132

A posterolateral fibular point determination unit 1321 is used for determining the fibular point for posterolateral structure reconstruction, which specifically includes: an anterolateral styloid process and a posteromedial edge.

A posterolateral tibial point determination unit 1322 is used for determining the tibial point for posterolateral structure reconstruction, which specifically includes: move a Gerdy tubercle inferior portion by a preset distance in a negative direction of a y-axis of the tibial coordinate system to point to a right posterior portion.

A posterolateral femoral point determination unit 1323 is used for determining the femoral point for posterolateral structure reconstruction, which specifically includes: use a method of moving a distal posterior side of a lateral epicondyle top point on the femur by preset distances in a negative direction of a y-axis and in a negative direction of a z-axis of the femoral coordinate system as a single-tunnel femoral point selection method; or use a method of moving the lateral epicondyle top point on the femur by a first preset distance towards a posterior side in a negative direction of a y-axis and a second preset distance towards a distal side in a negative direction of the z-axis of the femoral coordinate system as a double-tunnel femoral point selection method.

Further, in the case of the posterolateral tibial point determination unit 1322, a Gerdy tubercle inferior portion is moved by a preset distance in a negative direction of a y-axis of the tibial coordinate system to point to a right posterior portion, where the preset distance ranges from 9 mm to 11mm.

In the case of the posterolateral femoral point determination unit 1323, a method of moving a distal posterior side of a lateral epicondyle top point on the femur by preset distances in a negative direction of a y-axis and in a negative direction of a z-axis of the femoral coordinate system is used as a single-tunnel femoral point selection method, where the preset distances each range from 4 mm to 5mm; or a method of moving the lateral epicondyle top point on the femur by a first preset distance towards a posterior side in a negative direction of a y-axis, and a second preset distance towards a distal side in a negative direction of the z-axis of the femoral coordinate system is used as a double-tunnel femoral point selection method, where the first preset distance ranges from 5 mm to 7mm, and the second preset distance ranges from 9 mm to 11 mm.

### (3) Medial patellofemoral ligament reconstruction sub-module 133

A medial patellofemoral ligament femoral point determination unit 1331 is used for determining the femoral point for posteromedial patellofemoral ligament reconstruction, which specifically is an incenter of a triangle delimited by means of a medial epicondyle top point, an adductor tubercle and a gastrocnemius tubercle as the femoral point.

A medial patellofemoral ligament patellar point determination unit 1332 is used for determining the patellar point for patellofemoral ligament reconstruction, which specifically includes: move a superior patellar pole inferior portion by a preset distance in a positive direction of a patellar x-axis to set a start point for patellofemoral ligament single-tunnel reconstruction, where the preset distance is one third of a distance between a superior patellar pole and an inferior patellar pole; and by passing through an origin of the patellar coordinate system, make a perpendicular line of a plane parallel to the patellar x-axis and a patellar y-axis where a point at a patellar surface midline is located, thenset a point where the perpendicular line intersects the plane as an end point for patellofemoral ligament single tunnel reconstruction.

It should be noted that the ranges of the numbers listed above are the best preset distances selected as experience accumulates during actual surgery. During actual application, more proper preset distances may be selected according to actual situations or as experience continuously accumulates.

Moreover, in the cases of the fibular point, the femoral point, the tibial point and the patellar point, positions of the fibular point, the femoral point, the tibial point and the patellar point may be directly selected to determine a position of a channel; or a ligament attachment site footprint area boundary (obtained through multi-point selection or continuous drawing) is drawn by means of a probe under an arthroscope, then the fibular point, the femoral point, the tibial point and the patellar point are selected according to a required channel diameter, so as to determine the position of the reconstruction channel.

After the reconstruction channel is determined, the reconstruction channel is used as a pin entry channel for posteromedial structure, posterolateral structure and medial patellofemoral ligament reconstruction, and posteromedial structure, posterolateral structure and medial patellofemoral ligament grafts are grafted to reconstruct a posteromedial structure, a posterolateral structure and a medial patellofemoral ligament.

Further, the channel positioning system for posteromedial structure, posterolateral structure and medial patellofemoral ligament reconstruction further includes:
a reconstruction channel mechanical arm positioning device 2 for being connected to the reconstruction channel point collecting and modeling device to carry out physically assisted positioning on the reconstruction channel according to a determined specific channel position of the reconstruction channel, so as to guide posteromedial structure, posterolateral structure and medial patellofemoral ligament reconstruction surgery;
a reconstruction channel arthroscopy device 3 for observing an inner structure of a joint to undergo surgery, and monitoring posteromedial structure, posterolateral structure and medial patellofemoral ligament reconstruction surgery processes in real time, where the reconstruction channel point collecting and modeling device is used for positioning the specific channel position of the reconstruction channel according to the inner structure of the joint to undergo surgery.

Preferably, the reconstruction channel arthroscopy device 3 includes an arthroscope and a display device, and the display device is a display screen. The reconstruction channel arthroscopy device 3 includes a hollow rod, lenses, optical fibers and a photographing device are fixed in the hollow rod, the photographing device is connected to a display device, the hollow rod is connected to a power device, and an end of the hollow rod is guided into the joint by means of the power device;

The hollow rod is a thin rod having a length of more than 20 cm and a thickness ranging from 4 mm to 5 mm, which is used for being inserted into a joint cavity. A group of optical fibers and a group of lenses are contained in the rod, the optical fibers transmit light into a joint, and the lenses transmit out images in the joint. Outside the joint, the optical fibers are connected to a cold light source by means of an optical cable, such that the cold light source may illuminate the joint. The lenses are connected to a host and the display device 4 by means of a camera, so as to reflect images in the joint on the display device 4. The arthroscope is placed into the joint through a small incision about ranging from 0.8 mm to 1.0 cm in a skin, and the camera and a display apparatus are connected behind the arthroscope, such that morphology and lesions in the joint may be directly observed.

Preferably, the reconstruction channel point collecting and modeling device 1 includes a probe, a tracker and a computer, and the tracker is connected to the computer.

Preferably, the probe is used for collecting a plurality of sampling points under an arthroscope, the tracker transmits the sampling points to the computer, the plurality of sampling points form a sampling point set, and the computer establishes a three-dimensional coordinate system and a tunnel spatial path model according to the sampling point set, and determines positions of tibial and femoral tunnel inner orifices according to a predetermined algorithm.

Preferably, the reconstruction channel mechanical arm positioning device 2 includes a mechanical arm, and a hollow sleeve is arranged at an end of the mechanical arm. What can be added is that by means of the mechanical arm in the present disclosure, assisted positioning may be carried out to enable a drill bit and a guide pin to accurately drill into or be guided into a tunnel inner orifice for surgery : moreover, anesthesia may be carried out, a tourniquet is bound, positions (90-degree knee flexion and placement of a baffle on a lateral surface of a proximal section of a thigh) are arranged, a body surface is marked, disinfection and draping are carried out, whether a diagnostic arthroscope is suitable for mechanical arm assisted anterior cruciate ligament reconstruction surgery is determined again, an autograft is cut and prepared, and other operations before surgery may be carried out.

Preferably, the mechanical arm is a seven-degree-of-freedom mechanical arm with has high flexibility, and may rotate at a plurality of angles to align the hollow sleeve with the tunnel inner orifice.

Preferably, an inner diameter of the hollow sleeve is greater than an inner diameter of the tunnel, such that the drill bit and the guide pin may be inserted into the hollow sleeve and drill into the tunnel inner orifice for surgery.

After the tunnel position for ligament reconstruction is obtained by means of the above system, posteromedial structure, posterolateral structure or medial patellofemoral ligament grafts are grafted to reconstruct a posteromedial structure, a posterolateral structure and a medial patellofemoral ligament.

### Example 2

A positioning method for posteromedial structure, posterolateral structure and medial patellofemoral ligament reconstruction includes steps as follows:
S1: establish any one or more of a femoral coordinate system, a tibial coordinate system and a patellar coordinate system;
S2: select femoral feature points, tibial feature points, fibular feature points or patellar feature points by means of a probe under an arthroscope; and
S3: determine a femoral point, a tibial point, a fibular point or a patellar point during reconstruction according to a preset reconstruction algorithm on the basis of the femoral feature points, the tibial feature points, the fibular feature points or the patellar feature points, and further determine a specific channel position of a posteromedial structure, posterolateral structure or medial patellofemoral ligament reconstruction channel by means of the femoral point, the tibial point, the fibular point or the patellar point.
Further, the step S1 of establishing any one or more of a femoral coordinate system, a tibial coordinate system and a patellar coordinate system specifically includes:
S11: fix an optical femoral marker at a femur, fix an optical tibial marker at a tibia, or fix an optical patellar marker at a patella, so as to track a position of the femur, a position of the tibia or a position of the patella in real time by an optical tracker;
S12: obtain a femoral position data set of the optical femoral marker under the optical tracker, and compute a hip midpoint by fitting the femoral position data set;
S13: obtain a tibial position data set of the optical tibial marker under the optical tracker, fit the tibial position data set to obtain a plane, take a normal line of the plane as a flexion-extension axis of the tibia, and take the flexion-extension axis as a femoral x-axis of the femoral coordinate system and a tibial x-axis of the tibial coordinate system; and
S14: obtain position information of a knee midpoint and an ankle midpoint, take a connecting line between the knee midpoint and the hip midpoint as a femoral y-axis of the femoral coordinate system, establish a femoral z-axis by means of a cross product of the femoral x-axis and the femoral y-axis, and take the knee midpoint as an origin of the femoral coordinate sy stem, such that the femoral coordinate system is established; take a connecting line between the knee midpoint and the ankle midpoint as a tibial y-axis of the tibial coordinate system, establish a tibial z-axis by means of a cross product of the tibial x-axis and the tibial y-axis, and take the knee midpoint as an origin of the tibial coordinate system, such that the tibial coordinate system is established; and take a connecting line between a superior patellar pole and an inferior patellar pole as a patellar x-axis of the patellar coordinate system, take a connecting line between a patellar widest portion medial point and a patellar widest portion lateral point as a patellar y - axis of the patellar coordinate system, establish a patellar z-axis by means of a cross product of the patellar x-axis and the patellar y-axis, and take a point where the two connecting lines intersect as an origin of the patellar coordinate system, such that the patellar coordinate system is established.

Further, in the step S3, the preset reconstruction algorithm includes one or more of algorithms as follows:
a posteromedial structure reconstruction algorithm for reconstructing a specific channel position of a posteromedial structure reconstruction channel;
a posterolateral structure reconstruction algorithm for reconstructing a specific channel position of a posterolateral structure reconstruction channel;
a medial patellofemoral ligament reconstruction algorithm for reconstructing a specific channel position of a medial patellofemoral ligament reconstruction channel.

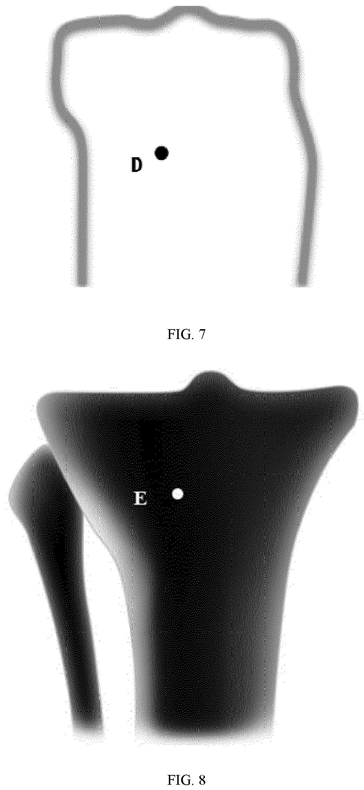

Further, the posteromedial structure reconstruction algorithm specifically includes:
determine the femoral point for posteromedial structure reconstruction, as shown in FIG. 6, which specifically includes: use a method of moving a medial epicondyle top point A on the femur by a first preset distance in a positive direction of a y-axis and a second preset distance in a negative direction of a z-axis of the femoral coordinate system as a double-tunnel femoral point selection method, use a method of moving the medial epicondyle top point on the femur by preset distances in a positive direction of a y-axis and in a negative direction of a z-axis of the femoral coordinate system as a single-tunnel femoral point selection method, or determine the femoral point including an adductor tubercle B proximal side and a femoral medial central established proximal side and
determine the tibial point for posteromedial structure reconstruction, as shown in FIGs. 7 and 8, which specifically includes: move a posteromedial tibial plateau inferior portion by a preset distance in a negative direction of a y-axis of the tibial coordinate system to point to a tibial anterolateral side, and move a Gerdy tubercle E, from a distal and posterior medial crest constriction point D anterior side, by a preset distance in a negative direction of a z-axis of the tibial coordinate system to point to a tibial lateral surface or a medial crest superior portion and a constriction point.

Further, the posteromedial structure reconstruction algorithm further includes:
the step of using, when the femoral point for posteromedial structure reconstruction is determined, a method of moving a medial epicondyle top point A on the femur by a first preset distance in a positive direction of a y-axis and a second preset distance in a negative direction of a z-axis of the femoral coordinate system as a double-tunnel femoral point selection method, where the first preset distance ranges from 4 mm to 5 mm, and the second preset distance ranges from 12 mm to 14 mm; and the step of using a method of moving the medial epicondyle top point A on the femur by preset distances in a positive direction of a y-axis and in a negative direction of a z-axis of the femoral coordinate system as a single-tunnel femoral point selection method, where the preset distances each ranges from 8 mm to 10 mm; and
the step of moving, when the tibial point for posteromedial structure reconstruction is determined, a posteromedial tibial plateau inferior portion by a preset distance in a negative direction of a y-axis of the tibial coordinate system to point to a tibial anterolateral side, where the preset distance ranges from 9 mm to 11mm; and the step of moving a Gerdy tubercle E, from a distal and posterior medial crest constriction point D anterior side, a preset distance in a negative direction of a z-axis of the tibial coordinate system to point to a tibial lateral surface or a medial crest superior portion and a constriction point, where the preset distance ranges from 9 mm to 11 mm.

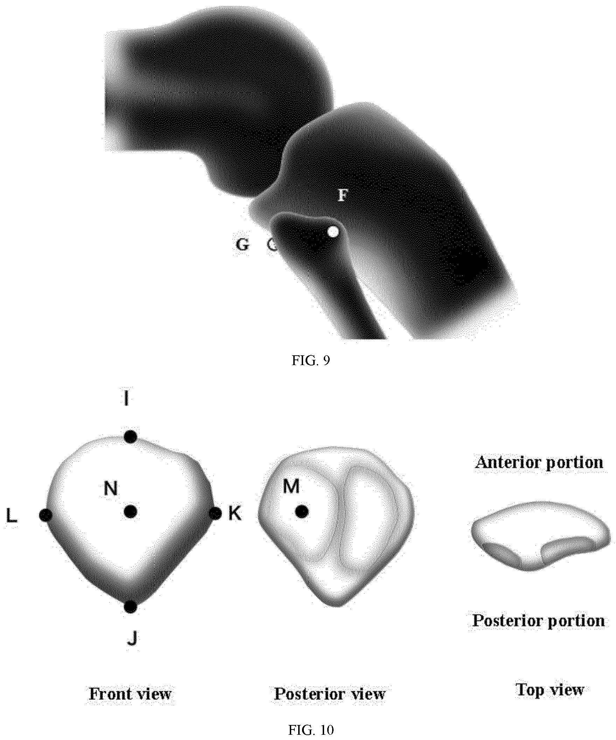

Further, the posterolateral structure reconstruction algorithm specifically includes:
determine the fibular point for posterolateral structure reconstruction, as shown in FIG. 9, which specifically includes: an anterolateral styloid process F and a posteromedial edge G;
determine the tibial point for posterolateral structure reconstruction, as shown in FIG. 8, which specifically includes: move a Gerdy tubercle E inferior portion by a preset distance in a negative direction of a y-axis of the tibial coordinate system to point to a right posterior portion; and
determine the femoral point for posterolateral structure reconstruction, as shown in FIG. 6, which specifically includes: use a method of moving a distal posterior side of a lateral epicondyle top point H on the femur by preset distances in a negative direction of a y-axis and in a negative direction of a z-axis of the femoral coordinate system as a single-tunnel femoral point selection method; or use a method of moving the lateral epicondyle top point on the femur by a first preset distance towards a posterior side in a negative direction of a y-axis and a second preset distance towards a distal side in a negative direction of the z-axis of the femoral coordinate system as a double-tunnel femoral point selection method.

Further, the posterolateral structure reconstruction algorithm further includes:
the step of moving, when the tibial point for posterolateral structure reconstruction is determined, a Gerdy tubercle E inferior portion by a preset distance in a negative direction of a y-axis of the tibial coordinate system to point to a right posterior portion, where the preset distance ranges from 9 mm to 11 mm; and
the step of using, when the femoral point for posterolateral structure reconstruction is determined, a method of moving a distal posterior side of a lateral epicondyle top point H on the femur by preset distances in a negative direction of a y-axis and in a negative direction of a z-axis of the femoral coordinate system as a single-tunnel femoral point selection method, where the preset distances each range from 4 mm to 5 mm; or the step of using a method of moving the lateral epicondyle top point on the femur by a first preset distance towards a posterior side in a negative direction of a y-axis and a second preset distance towards a distal side in a negative direction of the z-axis of the femoral coordinate system as a double-tunnel femoral point selection method, where the first preset distance ranges from 5 mm to 7 mm, and the second preset distance ranges from 9 mm to 11 mm.

Further, the medial patellofemoral ligament reconstruction algorithm specifically includes:
determine the femoral point for patellofemoral ligament reconstruction, as shown in FIG. 6, which specifically is an incenter of a triangle delimited by means of a medial epicondyle top point, an adductor tubercle and a gastrocnemius tubercle as the femoral point;
determine the patellar point for patellofemoral ligament reconstruction, as shown in FIG. 10, which specifically includes: move a superior patellar pole I inferior portion by a preset distance in a positive direction of a patellar x-axis to set a start point for patellofemoral ligament single-tunnel reconstruction, where the preset distance is one third of a distance between a superior patellar pole I and an inferior patellar pole J; and make, by passing through an origin of the patellar coordinate system , a perpendicular line of a plane parallel to the patellar x-axis and a patellar y-axis where a point N at a patellar surface midline is located, and set a point where the perpendicular line intersects the plane as an end point for patellofemoral ligament single tunnel reconstruction;
determine the medial patellofemoral ligament reconstruction tunnel, which specifically includes: determine a medial patellofemoral ligament single-tunnel reconstruction channel by means of the start point and the end point for patellofemoral ligament single-tunnel reconstruction; or translate the medial patellofemoral ligament single-tunnel reconstruction channel by preset distances in a positive direction and a negative direction of a patellar x-axis to determine a medial patellofemoral ligament double-tunnel reconstruction channel.

### Example 3

An intelligent control method for ligament reconstruction on the basis of the positioning system for posteromedial structure, posterolateral structure and medial patellofemoral ligament reconstruction in Example 1 includes steps as follows:
carry out preparation before surgery, specifically, according to patient' s medical history, specialized physical examination and megnetic resonace imaging (MRI) data, determine surgical indications and surgical methods, especially determine whether a patient is suitable for mechanical arm assisted anterior cruciate ligament reconstruction surgery.

Carry out operations before surgery, specifically, carry out anesthesia, bind a tourniquet, arrange positions (90-degree knee flexionand placement of a baffle on a lateral surface of a proximal section of a thigh), mark a body surface, carry out disinfection and draping, determine whether a diagnostic arthroscope is suitable for mechanical arm assisted anterior cruciate ligament reconstruction surgery again, and cut and prepare an autograft.

S100: Collect several position points within a sight range of a reconstruction channel arthroscopy device to form a sampling point set, establish a three-dimensional coordinate system according to the sampling point set, and according to a prompt of a point collecting and modeling device, select a medial meniscus most-medial side, a lateral meniscus most-lateral side, medial malleolus body surface positioning and lateral malleolus body surface positioning by means of a rigid probe under an arthroscope. An x-axis is determined by means of the medial meniscus most-medial side and the lateral meniscus most-lateral side, and a z-axis (along a long axis of a shank) is determined by making two perpendicular lines of the medial meniscus most-medial side and the lateral meniscus most-lateral side by passing through medial malleolus body surface positioning and lateral malleolus body surface positioning midpoints, such that a three-dimensional coordinate system is established.

S200: Select several bone feature points from the three-dimensional coordinate system, and establish a tunnel position path model according to the bone feature points; determine whether a surgical site is a left knee or a right knee on the system; select surgical methods, where in the case of anterior cruciate ligament single-bundle and double-bundle reconstruction surgery, there is no significantdifference in the selection of feature points; according to the different surgical methods, the positions of the tibial tunnel and femoral tunnel inner orifice were slightly different.

S300: Determine a position of a tunnel inner orifice according to the tunnel position path model;
simulate a positioning map, specifically, generate joint surface feature point and tunnel inner orifice position effect maps on a point collecting and modeling system interface; and generate anterior and lateral tunnel position effect maps, and determine that the several effect maps are basically consistent by a surgeon.

S400: Position a hollow sleeve by means of a mechanical arm according to the position of the tunnel inner orifice, so as to align an inner cavity of the hollow sleeve with the tunnel inner orifice.

S500: Establish a tunnel by means of a tool under guidance of the hollow sleeve.

Further, the step S200 includes:
S201: Firstly, carry out rough point collecting and modeling, specifically, carry out multi-point collecting on femoral and the tibial joint surfaces by means of a rigid probe respectively; simulate joint surface effect maps by a computer through multi-point collecting; and then transmit the joint effect maps to a display device by the computer to be displayed.

In the process of rough point collecting and modeling, feature points may be collected by means of a rigid probe under an arthroscope, or rectification may be carried out by means of a model three - dimensionally reconstructed according preoperative imaging data.
S202: Carry out fine point collecting to collect anterior cruciate femoral feature points including a top point and a lower reference point for anterior cruciate ligament reconstruction, anterior cruciate tibial feature points including a medial intercondylar crest lateral slope surface, a lateral meniscus anterior horn free edge, a transverse knee ligament, a lateral intercondylar crest top point, and a tibial plateau posterior cruciate ligament (PCL) anterior edge for anterior cruciate ligament reconstruction, posterior cruciate femoral feature points including a front reference point and a distal cartilage edge for posterior cruciate ligament reconstruction, and posterior cruciate tibial feature points including a joint capsule attachment point, a tibial PCL footprint half-area medial midpoint, and a tibial PCL footprint area lateral edge for posterior cruciate ligament reconstruction, and determine positions of tibial and femoral tunnel inner orifices by the computer according to a predetermined algorithm and fine point collecting.

Further, the step S500 includes:
S501: align the hollow sleeve with a tibial tunnel inner orifice, insert a drill bit into the hollow sleeve to drill a tibial tunnel outer orifice recess, and insert a guide pin into the tibial tunnel outer orifice recess to establish a tibial tunnel;
S502: align the hollow sleeve with a femoral tunnel inner orifice, insert the drill bit into the hollow sleeve to drill a femoral tunnel outer orifice recess, and insert the guide pin into the femoral tunnel outer orifice recess to establish a femoral tunnel.

A mechanical arm positioning step further includes: S600: after a tunnel direction is determined, make a mechanical arm in place according to a predetermined straight line. In this step, a surgeon is required to determine that a tunnel position is not shifted at any time. A specific implementation method is as follows: mark, by a surgeon by means of a rigid probe, a position of a tibial tunnel inner orifice and a position of a femoral tunnel inner orifice determined according to experience of the surgeon, and determine, through comparison, whether the positions are on the straight line determined by an axis of the hollow sleeve.

S601: If the surgeon is not satisfied with the above calibration result, abandon the above point taking method, and directly mark tibial and femoral tunnel inner orifices.

S602: If the surgeon is satisfied with the above calibration result, continue to carry out operations, that is, under the condition of knee fixation, drill a tibial tunnel outer orifice recess by a drill bit, and establish a tibial tunnel by a guide pin. After the tibial tunnel is established, whether the requirement that a tibial tunnel and a femoral tunnel are established at one time by one guide pin is satisfied is determined, and a femoral tunnel is established, so as to complete mechanical arm assisted surgical steps. In the case of accurate tibial tunnel establishment, after the drill bit drills a tunnel outer orifice recess, the guide pin may be drilled into the recess in a mechanical arm positioning direction or by means of existing surgical instruments including various positioners.

Similar to operations of conventional anterior cruciate ligament reconstruction surgery, later operations of the mechanical arm include: expand a tunnel by a drill bit, carry out intercondylar fovea plasty, graft and fix a graft, carry out biomechanical examination after grafting, carry out suture and binding up, carry out rehabilitation training, etc.

### Example 4

As shown in FIG. 9, a chuck-type patellar marking device is shown. Several (such as three) screws may be fixed on a patella, reflective marking balls may be mounted on the device to track a spatial position of the patella.

Selection of patellar feature points by a probe: as shown in FIG. 8, a superior patellar pole I, an inferior patellar pole J, a patellar widest portion medial point K, a patellar widest portion lateral point L, a point M at a patellar medial surface recession, a point N at a patellar surface midline are selecte d, and a computer determines a position of a tunnel inner orifice of the patella according to a medial patellar ligament reconstruction algorithm.

The patella is divided into three equal parts from top to bottom by points I and J (as shown in FIG. 10).

Medial patellar ligament single-tunnel reconstruction channel start point: a superior patellar pole I inferior portion is moved by a preset distance in a positive direction of a patellar x-axis to set the start point for patellar ligament single-tunnel reconstruction, where the preset distance is one third of a distance between a superior patellar pole and the inferior patellar pole.

Medial patellar ligament single-tunnel reconstruction end point: a perpendicular line of a plane parallel to the patellar x-axis and a patellar y-axis where a point N at a patellar surface midline is located is made, by passing through an origin of the patellar coordinate system, to set a point where the perpendicular line intersects the plane as an end point for patellar ligament single-tunnel reconstruction.

Determination of medial patellar ligament single-tunnel reconstruction channel: the medial patellar ligament single-tunnel reconstruction channel may be determined by means of the above start point and end point.

Determination of medial patellar ligament double-tunnel reconstruction channel: the medial patellar ligament single-tunnel reconstruction channel is translated by preset distances in a positive direction and a negative direction of a patellar x-axis, where the preset distances each may range from 4 mm to 6 mm.

What are mentioned above are merely preferred embodiments of the present disclosure, the scope of protection of the present disclosure is not limited to the above examples, and all technical solutions following the idea of the present disclosure fall within the scope of protection of the present disclosure. It should be noted that several improvements and modifications made by those of ordinary skill in the art without departing from the principle of the present disclosure should fall within the scope of protection of the present disclosure.

Various technical features of the above examples can be arbitrarily combined. In order to simplify description, all possible combinations of the various technical features of the above examples are not described. However, if only combinations of these technical features do not conflict, they shall be considered to fall within the scope of description of the present disclosure.

## Claims

1. A positioning system for posteromedial structure, posterolateral structure and medial patellofemoral ligament reconstruction, comprising:
a reconstruction channel point collecting and modeling device for determining a specific channel position of a reconstruction channel and specifically comprising:
a reconstruction channel coordinate system establishment module for establishing any one or more of a femoral coordinate system, a tibial coordinate system and a patellar coordinate system;
a reconstruction channel bone feature point obtainment module for selecting femoral feature points, tibial feature points, fibular feature points or patellar feature points by means of a probe under an arthroscope;
a reconstruction channel position determination module for determining a femoral point, a tibial point, a fibular point and a patellar point during reconstruction according to a preset reconstruction algorithm on the basis of the femoral feature points, the tibial feature points, the fibular feature points and the patellar feature points, and further determining a specific channel position of a posteromedial structure, posterolateral structure or medial patellofemoral ligament reconstruction channel by means of the femoral point, the tibial point, the fibular point and the patellar point.

2. The positioning system for posteromedial structure, posterolateral structure and medial patellofemoral ligament reconstruction according to claim 1, wherein the reconstruction channel coordinate system establishment module specifically comprises:
a position tracking unit for fixing an optical femoral marker, an optical tibial marker or an optical patellar marker on a femur, a tibia or a patella respectively, and tracking a position of the femur, a position of the tibia or a position of the patella in real time by an optical tracker;
a hip center fitting unit for obtaining a femoral position data set of the optical femoral marker under the optical tracker, and fitting the femoral position data set to compute a hip midpoint;
a flexion-extension axis obtainment unit for obtaining a tibial position data set of the optical tibial marker under the optical tracker, fitting the tibial position data set to obtain a plane, taking a normal line of the plane as a flexion-extension axis of the tibia, and taking the flexion-extension axis as a femoral x-axis of the femoral coordinate system and a tibial x-axis of the tibial coordinate system;
a coordinate axis determination unit for establishing the femoral coordinate system, the tibial coordinate system or the patellar coordinate system.

3. The positioning system for posteromedial structure, posterolateral structure and medial patellofemoral ligament reconstruction according to claim 1, wherein the reconstruction channel position determination module specifically comprises any one or more of sub-modules as follows:
a posteromedial structure reconstruction sub-module for executing a posteromedial structure reconstruction algorithm, so as to reconstruct a specific channel position of a posteromedial structure reconstruction channel;
a posterolateral structure reconstruction sub-module for executing a posterolateral structure reconstruction algorithm, so as to reconstruct a specific channel position of a posterolateral structure reconstruction channel;
a medial patellofemoral ligament reconstruction sub-module for executing a medial patellofemoral ligament reconstruction algorithm, so as to reconstruct a specific channel position of a medial patellofemoral ligament reconstruction channel.

4. The positioning system for posteromedial structure, posterolateral structure and medial patellofemoral ligament reconstruction according to claim 3, wherein the posteromedial structure reconstruction sub-module specifically comprises:
a posteromedial femoral point determination unit for determining the femoral point for posteromedial structure reconstruction, which specifically comprises: using a method of moving a medial epicondyle top point on the femur by a first preset distance in a positive direction of a y-axis and a second preset distance in a negative direction of a z-axis of the femoral coordinate system as a double-tunnel femoral point selection method, using a method of moving the medial epicondyle top point on the femur by preset distances in a positive direction of a y-axis and in a negative direction of a z-axis of the femoral coordinate system as a single-tunnel femoral point selection method, or determining the femoral point comprising an adductor tubercle proximal side and a femoral medial central established proximal side;
a posteromedial tibial point determination unit for determining the tibial point for posteromedial structure reconstruction, which specifically comprises: moving a posteromedial tibial plateau inferior portion by a preset distance in a negative direction of a y-axis of the tibial coordinate system to point to a tibial anterolateral side, and moving a Gerdy tubercle distal portion and a posterior medial crest constriction point anterior side by preset distances in a negative direction of a z-axis of the tibial coordinate system to point to a tibial lateral surface or a medial crest superior portion and a constriction point.

5. The positioning system for posteromedial structure, posterolateral structure and medial patellofemoral ligament reconstruction according to claim 4, further comprising:
the posteromedial femoral point determination unit, wherein in the using a method of moving a medial epicondyle top point on the femur by a first preset distance in a positive direction of a y-axis and a second preset distance in a negative direction of a z-axis of the femoral coordinate system as a double-tunnel femoral point selection method, the first preset distance ranges from 4 mm to 5mm, and the second preset distance ranges from 12 mm to 14 mm; and in the using a method of moving the medial epicondyle top point on the femur by preset distances in a positive direction of a y-axis and in a negative direction of a z-axis of the femoral coordinate system as a single-tunnel femoral point selection method, the preset distances each range from 8 mm to 10 mm;
the posteromedial tibial point determination unit, wherein in the moving a posteromedial tibial plateau inferior portion by a preset distance in a negative direction of a y-axis of the tibial coordinate system to point to a tibial anterolateral side, the preset distance ranges from 9 mm to 11mm; and in the moving a Gerdy tubercle distal portion and a posterior medial crest constriction point anterior side by preset distances in a negative direction of a z-axis of the tibial coordinate system to point to a tibial lateral surface or a medial crest superior portion and a constriction point, the preset distances each range from 9 mm to 11 mm.

6. The positioning system for posteromedial structure, posterolateral structure and medial patellofemoral ligament reconstruction according to claim 3, wherein the posterolateral structure reconstruction sub-module specifically comprises:
a posterolateral fibular point determination unit for determining the fibular point for posterolateral structure reconstruction, which specifically comprises: an anterolateral styloid process and a posteromedial edge;
a posterolateral tibial point determination unit for determining the tibial point for posterolateral structure reconstruction, which specifically comprises: moving a Gerdy tubercle inferior portion by a preset distance in a negative direction of a y-axis of the tibial coordinate system to point to a right posterior portion;
a posterolateral femoral point determination unit for determining the femoral point for posterolateral structure reconstruction, which specifically comprises: using a method of moving a distal posterior side of a lateral epicondyle top point on the femur by preset distances in a negative direction of a y-axis and in a negative direction of a z-axis of the femoral coordinate system as a single-tunnel femoral point selection method; or using a method of moving the lateral epicondyle top point on the femur by a first preset distance towards a posterior side in a negative direction of a y-axis and a second preset distance towards a distal side in a negative direction of the z-axis of the femoral coordinate system as a double-tunnel femoral point selection method.

7. The positioning system for posteromedial structure, posterolateral structure and medial patellofemoral ligament reconstruction according to claim 6, further comprising:
the posterolateral tibial point determination unit, wherein in the moving a Gerdy tubercle inferior portion by a preset distance in a negative direction of a y-axis of the tibial coordinate system to point to a right posterior portion, the preset distance ranges from 9 mm to 11mm;
the posterolateral femoral point determination unit, wherein in the using a method of moving a distal posterior side of a lateral epicondyle top point on the femur by preset distances in a negative direction of a y-axis and in a negative direction of a z-axis of the femoral coordinate system as a single-tunnel femoral point selection method, the preset distances each range from 4 mm to 5mm; or in the using a method of moving the lateral epicondyle top point on the femur by a first preset distance towards a posterior side in a negative direction of a y-axis and a second preset distance towards a distal side in a negative direction of the z-axis of the femoral coordinate system as a double-tunnel femoral point selection method, the first preset distance ranges from 5 mm to 7mm, and the second preset distance ranges from 9 mm to 11 mm.

8. The positioning system for posteromedial structure, posterolateral structure and medial patellofemoral ligament reconstruction according to claim 3, wherein the medial patellofemoral ligament reconstruction sub-module specifically comprises:
a medial patellofemoral ligament femoral point determination unit for determining the femoral point for patellofemoral ligament reconstruction, which specifically is an incenter of a triangle delimited by means of a medial epicondyle top point, an adductor tubercle and a gastrocnemius tubercle as the femoral point;
a medial patellofemoral ligament patellar point determination unit for determining the patellar point for patellofemoral ligament reconstruction, which specifically comprises: moving a superior patellar pole inferior portion by a preset distance in a positive direction of a patellar x-axis to set a start point for patellofemoral ligament single-tunnel reconstruction, wherein the preset distance is one third of a distance between a superior patellar pole and an inferior patellar pole; and making, by passing through an origin of the patellar coordinate system, a perpendicular line of a plane parallel to the patellar x-axis and a patellar y-axis where a point at a patellar surface midline is located, and setting a point where the perpendicular line intersects the plane as an end point for patellofemoral ligament single tunnel reconstruction.

9. The positioning system for posteromedial structure, posterolateral structure and medial patellofemoral ligament reconstruction according to claim 1, further comprising:
a reconstruction channel mechanical arm positioning device for being connected to the reconstruction channel point collecting and modeling device to carry out physically assisted positioning on the reconstruction channel according to a determined specific channel position of the reconstruction channel, so as to guide posteromedial structure, posterolateral structure and medial patellofemoral ligament reconstruction surgery.

10. The positioning system for posteromedial structure, posterolateral structure and medial patellofemoral ligament reconstruction according to claim 1, further comprising:
a reconstruction channel arthroscopy device for observing an inner structure of a joint to undergo surgery, and monitoring posteromedial structure, posterolateral structure and medial patellofemoral ligament reconstruction surgery processes in real time, wherein the reconstruction channel point collecting and modeling device is used for positioning the specific channel position of the reconstruction channel according to the inner structure of the joint to undergo surgery.

11. The positioning system for posteromedial structure, posterolateral structure and medial patellofemoral ligament reconstruction according to claim 10, further comprising:
the reconstruction channel arthroscopy device comprising a hollow rod, wherein lenses, optical fibers and a photographing device are fixed in the hollow rod, the photographing device is connected to a display device, the hollow rod is connected to a power device, and an end of the hollow rod is guided into the joint by means of the power device;
the reconstruction channel point collecting and modeling device comprising the probe, a tracker and a computer, wherein the tracker is connected to the computer, the probe is used for collecting a plurality of sampling points under the arthroscope, the tracker transmits the sampling points to the computer, the plurality of sampling points form a sampling point set, and the computer establishes a three-dimensional coordinate system and a tunnel spatial path model according to the sampling point set, and determines positions of tibial and femoral tunnel inner orifices according to a predetermined algorithm;
the reconstruction channel mechanical arm positioning device comprising a mechanical arm, wherein a hollow sleeve is arranged at an end of the mechanical arm, the mechanical arm is a seven-degree-of-freedom mechanical arm, and an inner diameter of the hollow sleeve is greater than an inner diameter of a tunnel.

12. A positioning method for posteromedial structure, posterolateral structure and medial patellofemoral ligament reconstruction, comprising steps as follows:
S1: establishing any one or more of a femoral coordinate system, a tibial coordinate system and a patellar coordinate system;
S2: selecting femoral feature points, tibial feature points, fibular feature points or patellar feature points by means of a probe under an arthroscope;
S3: determining a femoral point, a tibial point, a fibular point or a patellar point during reconstruction according to a preset reconstruction algorithm on the basis of the femoral feature points, the tibial feature points, the fibular feature points or the patellar feature points, and further determining a specific channel position of a posteromedial structure, posterolateral structure or medial patellofemoral ligament reconstruction channel by means of the femoral point, the tibial point, the fibular point or the patellar point.

13. The positioning method for posteromedial structure, posterolateral structure and medial patellofemoral ligament reconstruction according to claim 12, wherein the step S1 of establishing any one or more of a femoral coordinate system, a tibial coordinate system and a patellar coordinate system specifically comprises:
S11: fixing an optical femoral marker at a femur, fixing an optical tibial marker at a tibia, or fixing an optical patellar marker at a patella, so as to track a position of the femur, a position of the tibia or a position of the patella in real time by an optical tracker;
S12: obtaining a femoral position data set of the optical femoral marker under the optical tracker, and computing a hip midpoint by fitting the femoral position data set;
S13: obtaining a tibial position data set of the optical tibial marker under the optical tracker, fitting the tibial position data set to obtain a plane, taking a normal line of the plane as a flexion-extension axis of the tibia, and taking the flexion-extension axis as a femoral x-axis of the femoral coordinate system and a tibial x-axis of the tibial coordinate system;
S14: obtaining position information of a knee midpoint and an ankle midpoint, taking a connecting line between the knee midpoint and the hip midpoint as a femoral y-axis of the femoral coordinate system, establishing a femoral z-axis by means of a cross product of the femoral x-axis and the femoral y-axis, and taking the knee midpoint as an origin of the femoral coordinate system, such that the femoral coordinate system is established; taking a connecting line between the knee midpoint and the ankle midpoint as a tibial y -axis of the tibial coordinate system, establishing a tibial z-axis by means of a cross product of the tibial x-axis and the tibial y-axis, and taking the knee midpoint as an origin of the tibial coordinate system, such that the tibial coordinate system is established; and taking a connecting line between a superior patellar pole and an inferior patellar pole as a patellar x-axis of the patellar coordinate system, taking a connecting line between a patellar widest portion medial point and a patellar widest portion lateral point as a patellar y-axis of the patellar coordinate system, establishing a patellar z-axis by means of a cross product of the patellar x-axis and the patellar y-axis, and taking a point where the two connecting lines intersect as an origin of the patellar coordinate system, such that the patellar coordinate system is established.

14. The positioning method for posteromedial structure, posterolateral structure and medial patellofemoral ligament reconstruction according to claim 12, wherein in the step S3, the preset reconstruction algorithm comprises one or more of algorithms as follows:
a posteromedial structure reconstruction algorithm for reconstructing a specific channel position of a posteromedial structure reconstruction channel;
a posterolateral structure reconstruction algorithm for reconstructing a specific channel position of a posterolateral structure reconstruction channel;
a medial patellofemoral ligament reconstruction algorithm for reconstructing a specific channel position of a medial patellofemoral ligament reconstruction channel.

15. The positioning method for posteromedial structure, posterolateral structure and medial patellofemoral ligament reconstruction according to claim 14, wherein the posteromedial structure reconstruction algorithm specifically comprises:
determining the femoral point for posteromedial structure reconstruction, which specifically comprises: using a method of moving a medial epicondyle top point on the femur by a first preset distance in a positive direction of a y-axis and a second preset distance in a negative direction of a z-axis of the femoral coordinate system as a double-tunnel femoral point selection method, using a method of moving the medial epicondyle top point on the femur by preset distances in a positive direction of a y-axis and in a negative direction of a z-axis of the femoral coordinate system as a single-tunnel femoral point selection method, or determining the femoral point comprising an adductor tubercle proximal side and a femoral medial central established proximal side;
determining the tibial point for posteromedial structure reconstruction, which specifically comprises: moving a posteromedial tibial plateau inferior portion by a preset distance in a negative direction of a y-axis of the tibial coordinate system to point to a tibial anterolateral side, and moving a Gerdy tubercle distal portion and a posterior medial crest constriction point anterior side by preset distances in a negative direction of a z-axis of the tibial coordinate system to point to a tibial lateral surface or a medial crest superior portion and a constriction point.

16. The positioning method for posteromedial structure, posterolateral structure and medial patellofemoral ligament reconstruction according to claim 15, wherein the posteromedial structure reconstruction algorithm further comprises:
the using, when the femoral point for posteromedial structure reconstruction is determined, a method of moving a medial epicondyle top point on the femur by a first preset distance in a positive direction of a y-axis and a second preset distance in a negative direction of a z-axis of the femoral coordinate system as a double-tunnel femoral point selection method, wherein the first preset distance ranges from 4 mm to 5 mm, and the second preset distance ranges from 12 mm to 14 mm; and the using a method of moving the medial epicondyle top point on the femur by preset distances in a positive direction of a y-axis and in a negative direction of a z-axis of the femoral coordinate system as a single-tunnel femoral point selection method, wherein the preset distances each ranges from 8 mm to 10 mm;
the moving, when the tibial point for posteromedial structure reconstruction is determined, a posteromedial tibial plateau inferior portion by a preset distance in a negative direction of a y-axis of the tibial coordinate system to point to a tibial anterolateral side, wherein the preset distance ranges from 9 mm to 11mm; and the moving a Gerdy tubercle distal portion and a posterior medial crest constriction point anterior side by preset distances in a negative direction of a z-axis of the tibial coordinate system to point to a tibial lateral surface or a medial crest superior portion and a constriction point, wherein the preset distances each range from 9 mm to 11 mm.

17. The positioning method for posteromedial structure, posterolateral structure and medial patellofemoral ligament reconstruction according to claim 14, wherein the posterolateral structure reconstruction algorithm specifically comprises:
determining the fibular point for posterolateral structure reconstruction, which specifically comprises: an anterolateral styloid process and a posteromedial edge;
determining the tibial point for posterolateral structure reconstruction, which specifically comprises: moving a Gerdy tubercle inferior portion by a preset distance in a negative direction of a y-axis of the tibial coordinate system to point to a right posterior portion;
determining the femoral point for posterolateral structure reconstruction, which specifically comprises: using a method of moving a distal posterior side of a lateral epicondyle top point on the femur by preset distances in a negative direction of a y-axis and in a negative direction of a z-axis of the femoral coordinate system as a single-tunnel femoral point selection method; or using a method of moving the lateral epicondyle top point on the femur by a first preset distance towards a posterior side in a negative direction of a y-axis and a second preset distance towards a distal side in a negative direction of the z-axis of the femoral coordinate system as a double-tunnel femoral point selection method.

18. The positioning method for posteromedial structure, posterolateral structure and medial patellofemoral ligament reconstruction according to claim 17, wherein the posterolateral structure reconstruction algorithm further comprises:
the moving, when the tibial point for posterolateral structure reconstruction is determined, a Gerdy tubercle inferior portion by a preset distance in a negative direction of a y-axis of the tibial coordinate system to point to a right posterior portion, wherein the preset distance ranges from 9 mm to 11 mm;
the using, when the femoral point for posterolateral structure reconstruction is determined, a method of moving a distal posterior side of a lateral epicondyle top point on the femur by preset distances in a negative direction of a y-axis and in a negative direction of a z-axis of the femoral coordinate system as a single-tunnel femoral point selection method, wherein the preset distances each range from 4 mm to 5 mm; or the using a method of moving the lateral epicondyle top point on the femur by a first preset distance towards a posterior side in a negative direction of a y-axis and a second preset distance towards a distal side in a negative direction of the z-axis of the femoral coordinate system as a double-tunnel femoral point selection method, wherein the first preset distance ranges from 5 mm to 7 mm, and the second preset distance ranges from 9 mm to 11 mm.

19. The positioning method for posteromedial structure, posterolateral structure and medial patellofemoral ligament reconstruction according to claim 14, wherein the medial patellofemoral ligament reconstruction algorithm specifically comprises:
determining the femoral point for patellofemoral ligament reconstruction, which specifically is an incenter of a triangle delimited by means of a medial epicondyle top point, an adductor tubercle and a gastrocnemius tubercle as the femoral point;
determining the patellar point for patellofemoral ligament reconstruction, which specifically comprises: moving a superior patellar pole inferior portion by a preset distance in a positive direction of a patellar x-axis to set a start point for patellofemoral ligament single-tunnel reconstruction, wherein the preset distance is one third of a distance between a superior patellar pole and an inferior patellar pole; and making, by passing through an origin of the patellar coordinate system, a perpendicular line of a plane parallel to the patellar x-axis and a patellar y-axis where a point at a patellar surface midline is located, and setting a point where the perpendicular line intersects the plane as an end point for patellofemoral ligament single tunnel reconstruction;
determining the medial patellofemoral ligament reconstruction tunnel, which specifically comprises: determining a medial patellofemoral ligament single-tunnel reconstruction channel by means of the start point and the end point for patellofemoral ligament single-tunnel reconstruction; or translating the medial patellofemoral ligament single-tunnel reconstruction channel by preset distances in a positive direction and a negative direction of a patellar x-axis to determine a medial patellofemoral ligament double-tunnel reconstruction channel.

20. An intelligent control method for ligament reconstruction on the basis of the positioning system for posteromedial structure, posterolateral structure and medial patellofemoral ligament reconstruction of any one of claims 1-11, comprising steps as follows:
S100: collecting several position points within a sight range of a reconstruction channel arthroscopy device to form a sampling point set, and establishing a three-dimensional coordinate system according to the sampling point set;
S200: selecting several bone feature points from the three-dimensional coordinate system, and establishing a tunnel position path model according to the bone feature points;
S300: determining a position of a tunnel inner orifice according to the tunnel position path model;
S400: positioning a hollow sleeve by means of a mechanical arm according to the position of the tunnel inner orifice, so as to align an inner cavity of the hollow sleeve with the tunnel inner orifice;
S500: establishing a tunnel by means of a tool under guidance of the hollow sleeve.

21. The intelligent control method according to claim 20, wherein the step S200 comprises:
S201: carrying out multi-point collecting on femoral and tibial joint surfaces by means of a rigid probe, and simulating joint surface effect maps by a computer through multi-point collecting;
S202: carrying out fine point collecting to collect femoral feature points comprising a medial epicondyle top point, an adductor tubercle, a gastrocnemius tubercle and a lateral epicondyle top point, tibial feature points comprising a posterior medial crest constriction point and a Gerdy tubercle, fibular feature points comprising an anterolateral styloid process and a posteromedial edge, and patellar feature points comprising a superior patellar pole, an inferior patellar pole, a patellar widest portion medial point, a patellar widest portion lateral point, a point at a patellar medial surface recession and a point at a patellar surface midline, and determining positions of tibial, femoral and patellar tunnel inner orifices by the computer according to a predetermined algorithm and fine point collecting.

22. The intelligent control method according to claim 20, wherein the step S500 comprises:
S501: aligning the hollow sleeve with a tibial tunnel inner orifice, inserting a drill bit into the hollow sleeve to drill a tibial tunnel outer orifice recess, and inserting a guide pin into the tibial tunnel outer orifice recess to establish a tibial tunnel;
S502: aligning the hollow sleeve with a femoral tunnel inner orifice, inserting the drill bit into the hollow sleeve to drill a femoral tunnel outer orifice recess, and inserting the guide pin into the femoral tunnel outer orifice recess to establish a femoral tunnel.
